# EUROPÄISCHE PATENTANMELDUNG

(11) **EP 1 544 617 A2**
(43) Veröffentlichungstag der Anmeldung: **22.06.2005**
(21) Anmeldenummer: 04104548.5
(22) Anmeldetag: 21.09.2004
(51) Int. Cl.: G01N 33/574, C12Q 1/68, A61P 35/00, A61K 39/395, A61K 48/00, A61K 49/00, C07K 14/00

(54) **Verwendung von an Nifie 14 bindenden Substanzen zur Diagnose und Behandlung von Krebs**

(30) Priorität: 22.09.2003 DE 10345010
(71) Anmelder: Hinzmann, Bernd, Dr., 13127 Berlin (DE); Rosenthal, André, Dr., 14482 Potsdam (DE); Heiden, Esmeralda, Dr., 10589 Berlin (DE); Hermann, Klaus, Dr., 12679 Berlin (DE)
(72) Erfinder: Hinzmann, Bernd, Dr., 13127 Berlin (DE); Rosenthal, André, Dr., 14482 Potsdam (DE); Heiden, Esmeralda, Dr., 10589 Berlin (DE); Hermann, Klaus, Dr., 12679 Berlin (DE)

(57) **Zusammenfassung**

Die Erfindung betrifft Verwendungen von Nifie14 zur Diagnose und Behandlung von Krebs, sowie zum Screenen nach Substanzen für solche Zwecke.

## Beschreibung

### Technical field

Die Erfindung betrifft Verwendungen von Nifie14 oder daraus abgeleiteten Sequenzen zum Screenen nach daran bindenden Substanzen, die Verwendung von an Nifie14 bindenden Substanzen zur Diagnose und/oder Behandlung von Krebs, insbesondere von Kolon-, Rectum-, Lungen-, Magen-, Brust-, Ovar-, Nieren-, und/oder Uterustumoren. Die Erfindung betrifft des weiteren Nifie14 Sequenzen und Teilsequenzen.

### Background art

### Hintergrund der Erfindung und Stand der Technik

Das humane Gen Nifie14 kodiert für ein Protein, das aufgrund seiner Sequenzähnlichkeit zur Gruppe der G-Protein gekoppelten Rezeptoren (GPCRs) zugeordnet werden kann. Da es bislang molekularbiologisch, genetisch und biochemisch nicht experimentell charakterisiert worden ist, muß es zur Gruppe der ligandenlosen GPCRs, den sogenannten "orphan GPCRs" gerechnet werden. Weder das Gen, noch die mRNA, noch das Protein sind in wissenschaftlichen Publikationen charakterisiert worden.

Die Diagnose- und Therapiemöglichkeiten der eingangs genannten Tumorerkrankungen sind nur von eingeschränktem Nutzen. Gerade Tumore späterer Stadien mit einem aggressiven Wachstum, die bereits die Lymphknoten befallen haben oder bereits Metastasen gebildet haben, sind mit bestehenden Therapieformen nur unzureichend behandelbar. In vielen Patientengruppen liegen die 5-Jahres Überlebensraten unter 10%: Zudem besitzen viele der bekannten Therapeutika starke Nebenwirkungen.

In der Diagnostik stellt sich regelmäßig das Problem, kleinste Tumorzellpopulationen im Körper nachzuweisen. Dies ist sowohl im Rahmen der Früherkennung als auch bei der Nachsorge, beispielsweise nach chirurgischer Entfernung eines Hauptumors zur Detektion von eventuellen Mikrometastasen, wichtig.

Daher ist es wünschenwert, verbesserte Ansätze zur Diagnose und Therapie von Krebserkrankungen zur Verfügung zu stellen.

### Brief Description of Drawings

Fig. 1: Chipanalyse zur differentiellen Expression von Nifie14 in Kolontumorgewebe,

Fig. 2: Nifie14 Expression in Normalgeweben,

Fig. 3: Krebsgewebe Profiling (CPA),

Fig. 4: Tabellarische Auswertung des Profiling (CPA) von Fig. 3,

Fig. 5: Taqman Resultate zur Nifie14 Expression in Kolontumoren, bezogen auf zugeordnetes Normalgewebe,

Fig. 6: Taqman Resultate zur Nifie14 Expression in verschiedenen Normalgeweben, bezogen auf Kolon Normalgewebe,

Fig. 7: in situ Hybridisierung von Kolon Tumorgeweben.
Mode(s) for carrying out the invention

### Technisches Problem der Erfindung

Der Erfindung liegt daher das technische Problem zugrunde, pharmazeutische Zusammensetzungen zur Diagnose, auch zur Verlaufs- bzw. Progressionsprognose, und/oder zur Behandlung von Krebs, insbesondere der eingangs genannten Tumore, anzugeben sowie Mittel zu deren Identifizierung.

Grundzüge der Erfindung und bevorzugte Ausführungsformen.

Zur Lösung dieses technischen Problems lehrt die Verwendung einer für Nifie14 codierenden Nukleinsäure und/oder eines Nifie14 Peptids oder Proteins zur Detektion von Krebs oder zur Detektion eines Risikos der Erkrankung an Krebs oder zur Detektion eines Risikos einer Progression einer Krebserkrankung, wobei eine Gewebeprobe (des zu untersuchenden Gewebetypes bzw. Krebstypes, beispielsweise Ovartumor) auf Transkription oder Übertranskription von Nifie14 RNA oder auf Expression oder Überexpression eines Nifie14 Proteins untersucht wird. Eine an für Nifie14 codierende Nukleinsäure oder eine an Nifie14 Protein oder Peptid bindende Detektorsubstanz, vorzugsweise enthaltend eine Reportergruppe, kann verwendet werden, wobei Bindung besagter Nukleinsäure und/oder besagten Proteins oder Peptids an die Detektorsubstanz halbquantitativ oder quantitativ detektiert wird. In diesem Zusammenhang lehrt die Erfindung weiterhin ein Testsystem zur (in vitro) Detektion eines eingangs genannten Karzinoms oder eines Risikos der Erkrankung hieran oder der Progressionsprognose, enthaltend Mittel zur quantitativen Messung der Expression von Nifie14 in Gewebeproben, wobei diese Mittel beispielsweise Mittel zur Amplifikation und spezifischen Detektion von Nifie14 RNA und/oder eine Detektorsubstanz, insbesondere spezifisch für Nifie14 Protein, sein können.

Die Erfindung lehrt weiterhin die Verwendung einer Nifie14 RNA oder eines Nifie14 Proteins oder Peptids zum Screenen nach daran bindenden Substanzen, insbesondere prospektiven Wirkstoffen zur Modulierung, insbesondere Inhibierung, von besagter RNA oder besagtem Protein oder Peptid, oder prospektiven Detektorsubstanzen, wobei eine prospektive Substanz oder eine Mischung solcher prospektiver Substanzen mit besagter RNA oder besagtem Protein oder Peptid kontaktiert wird, wobei mit einem Bindungsassay Bindungsereignisse festgestellt werden, und wobei eine bindende prospektive Substanz, ggf. nach Dekonvolutierung, selektiert wird. In diesen Zusammenhängen lehrt die Erfindung weiterhin ein Screeningsystem zur Ermittlung von für die Behandlung von vorstehenden Tumorerkrankungen geeigneten Wirksubstanzen enthaltend eine Nifie14 Nukleinsäure oder ein Nifie14 Protein bzw. Peptid, Mittel zur Bestimmung von (in vitro) Bindungsereignissen an die Nifie14 Nukleinsäure oder an das Nifie14 Protein bzw. Peptid, und/oder Mittel zur Bestimmung der (in vitro) Aktivität von Nifie14 Protein. Hierbei kann Nifie14 in einem zellfreien oder einem zellbasierten System, letzteres insbesondere aufweisend Zellen aus vorstehend genannten Geweben bzw. eine hieraus entwickelte Zelllinie, vorliegen. Mittel zur Bestimmung von Bindungsereignissen können beispielsweise natürlicherweise in normalen oder in Tumorzellen z.B. an Nifie14 Protein bindende Substanzen bzw. Assoziationspartner umfassen, wobei über deren (freie) Konzentration bzw. Konzentrationsänderung bei Zugabe prospektiver Wirksubstanzen und/oder Detektorsubstanzen eine kompetitive Bindung einer bindenden Wirk- oder Detektorsubstanz bestimmt wird. Solche Mittel können aber auch physikalische bzw. physikalisch-chemische Methoden umfassen, wie beispielsweise Röntgenstrukturanalyse und/oder NMR, insbesondere zweidimensionale 1H/1H oder 15N/1H oder 14C/1H Korrelationsspektroskopie. Hierbei werden Spektren vor und nach der Zugabe einer prospektiven Wirk- oder Detektorsubstanz miteinander verglichen und im Falle von Änderungen ist ein Bindungsereignis festgestellt. Es kann mit Spektren oder dergleichen entweder von Nifie14 oder der prospektiven Substanz oder mit einer Kombination aus beidem gearbeitet werden. Selbstverständlich sind auch alle anderen fachüblichen Methoden der Bestimmung von Bindungsereignissen und/oder Proteinaktivitäten einsetzbar. Beispielsweise kann eine prospektive Substanz (oder mehrere Substanzen, räumlich voneinander getrennt) immobilisiert sein, wobei dann markiertes Nifie14 aufgetragen wird. Ein Bindungsereignis wird dann nach Auftrag und folgender Spülung durch Detektion, ggf. ortlich aufgelöst, der Markierung gebundenen Nifie14 festgestellt. Bindungsereignisse können auch ohne Markierung eines der Bindungspartner mittels der dem Fachmann geläufigen Biacore Technologie (Oberflächenplasmonen Resonanz) detektiert werden. Umgekehrt kann Nifie14 immobilisiert sein und es wird eine markierte prospektive Substanz oder eine Mischung hieraus aufgetragen. Bindungsereignisse werden analog der vorstehenden Variante festgestellt.

Die Erfindung lehrt schließlich die Verwendung einer Nifie14 inhibierenden oder daran bindenden Substanz zur Herstellung einer pharmazeutischen Zusammensetzung zur Behandlung und/oder Diagnose von Krebs, insbesondere eines der eingangs genannten Tumore.

Die Substanz kann ein Antikörper sein, welcher durch Immunisierung eines nicht-menschlichen Säugetiers mit einem Nifie14 Peptid oder Protein, mit hierfür codierender cDNA transfizierte Zellen, cDNA Immunisierung (Genovac), mit endogen ein solches Peptid oder Protein exprimierenden Tumorzellen, oder mit rekombinant hergestellten Nifie14 Peptiden oder Proteinen, erhältlich ist, oder ein Phage-Display-Antikörper sein. Es kann sich bei Immunogen auch um Membranvesikeln vorstehender Zellen handeln. Die Substanz kann aber auch eine Mimikryverbindung eines Antikörpers gegen ein Nifie14 Peptid oder Protein sein. Die Substanz kann schließlich ein Aptamer, eine antisense RNA, ein Ribozym oder eine siRNA gegen Nifie14 Nukleinsäuren sein. Aptemere können selbstverständlich gegen beliebige Moleküle, insbesondere Proteine, Enzyme usw., gerichtet sein. Die Substanz kann zusätzlich eine zytotoxische und/oder immunstimulierende Komponente tragen.

Bevorzugt ist es, wenn die vorstehenden, an Nifie14 Protein bindenden Substanzen in der Verwendung zu therapeutischen Zwecken spezifisch an das Nifie14 Protein binden und es in seiner biologischen Aktivität modulieren. Dies ist nicht erforderlich im Falle der Fusion bzw. Verbindung der Substanz mit einer zytotoxischen Komponente. Dies ist weiterhin nicht erforderlich, wenn die Substanz der Gewinnung eines anti-idiotypischen Antikörpers dient, welcher vom Immunsystem eines Patienten aufgrund seiner nicht-humanisierten Form als körperfremd erkannt wird und dem Immunsystem ansonsten ein Nifie14-Antigen präsentiert.

Die pharmazeutische Zusammensetzung kann zur beliebigen Applikation, beispielsweise i.v. oder i.p. Injektion, hergerichtet sein. Eine Herrichtung zur lokalen Applikation in Tumorzellen enthaltendem Gewebe wird sich empfehlen im Falle des Einsatzes einer zytotoxischen Komponente.

Die Erfindung läßt sich im Rahmen eines Verfahrens zur Diagnose bzw. (Progressions-) Prognose einer Tumorerkrankung verwenden, wobei eine Detektorsubstanz in einer Ausführungsform mit einer Reportergruppe in zu untersuchendes Gewebe, ggf. in vitro nach Gewebeentnahme, appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als Tumorzellen enthaltend qualifiziert bzw. als progressionsgefährdet oder nicht progressionsgefährdet eingestuft wird, sowie eines Verfahrens zur Behandlung einer Krebserkrankung, wobei eine erfindungsgemäße pharmazeutische Zusammensetzung in einer physiologisch wirksamen Dosis einem Patienten dargereicht wird. Im Falle der Diagnose bzw. Progressionsprognose kann zusätzlich oder alternativ eine Gewebeprobe mit einem erfindungsgemäßen Testsystem auf Nifie14 Expression untersucht werden.

Die Erfindung beruht insbesondere auf der Erkenntnis, daß Nifie14 in diversen Geweben unterschiedlich exprimiert wird, i.e. in den Tumorgeweben ist die Expression im Falle solcher Karzinome höher, verglichen mit normalen Zellen gleichen Gewebes und der daraus herleitbaren technische Lehre, daß Nifie14 als Zielmolekül bei der Diagnostik und Therapie bzw. Prophylaxe eingangs genannter Tumorerkrankungen eingesetzt werden kann. Nifie14 kann also als spezifischer Marker zur Identifizierung von Tumorzellen in den besagten Tumorgeweben dienen. Es ist erkannt worden, dass pathologisch normale Zellen mit einer erhöhten Expression ein erhöhtes Risiko aufweisen, zu Krebszellen zu transformieren. Auf der anderen Seite bietet die Inhibierung von Nifie14 die Möglichkeit, in die Tumor-spezifischen Nifie14 Assoziationen mit anderen Prozessen in den Tumorzellen einzugreifen und somit letztendlich den tumorzellenspezifisch veränderten Stoffwechsel zu stören und zu einem Absterben oder zumindest einer Wachstumshemmung der Tumorzellen, insbsondere aber einer Hemmung der Transformation zu Tumorzellen, beizutragen.

Im Rahmen der Erfindung kann es sich empfehlen, im Vorfeld einer Behandlung mit einer erfindungsgemäßen pharmazeutischen Zusammensetzung eine Probe aus einem Gewebe, welches als Tumorgewebe mit anderen Methoden identifiziert ist, zu entnehmen und die Gewebeprobe auf Expression bzw. Überexpression von Nifie14 zu untersuchen. Alternativ kann mit einer erfindungsgemäßen Detektorsubstanz zur Diagnose in vivo auf Nifie14 Abhängigkeit getestet werden. Wird eine Expression bzw. Überexpression von Nifie14 gegenüber Normalgewebe gleichen Typs festgestellt, so ist die Anwendung der erfindungsgemäßen pharmazeutischen Zusammensetzung indiziert.

Generell ist es im Rahmen der Erfindung möglich, patientenspezifisch auf differentielle Expression zu untersuchen, wobei Normalgewebeprobe und Tumorgewebeproben bzw. tumorverdächtige Gewebeproben dem (gleichen) Patienten entnommen und vergleichend auf Werte der Nifie14 Expression untersucht werden.

Handelt es sich bei dem Tumor um einem Typus, bei welchem Tumorzellen Nifie14 exprimieren, Normalzellen gleichen Gewebetyps jedoch nicht, so ist es besonders bevorzugt, wenn die an Nifie14 bindende Substanz zusätzlich eine zytotoxische und/oder immunstimulierende Komponente trägt. Dies führt dann letztendlich dazu, dass praktisch ausschließlich Tumorzellen getötet werden, sei es durch die Zytotoxizität, sei es durch Angriff durch das stimulierte Immunsystem, während Normalzellen in dem Gewebe praktisch vollständig erhalten bleiben. In dieser Ausführungsform braucht die bindende Substanz selbst nicht inhibierend auf Nifie14 zu wirken, da die bindende Substanz dann lediglich als Marker funktionieren muß, welcher die Komponenten zu Ziel-Tumorzellen trägt. Im Falle des Einsatzes einer zytotoxischen Komponente wird es sich besonders empfehlen, wenn die pharmazeutische Zusammensetzung zur lokalen Applikation in Tumorzellen enthaltendem Gewebe hergerichtet ist, beispielsweise zur Injektion.

Schließlich betrifft die Erfindung eine Nifie14 Nukleinsäure (Seq.-ID 1) oder ein Nifie14 Protein oder Peptid (Seq.-ID 2) sowie Teilsequenzen aus diesen Sequenzen sowie deren Verwendungen im vorstehend erläuterten Sinne.

Definitionen und weitere Ausführungsformen der Erfindung.

Nifie14 Sequenzen sind in den Seq.-ID 1 und 2 (siehe auch die Figuren) dargestellt. Die im Rahmen der Erfindung nutzbaren Nukleinsäuren oder Peptide oder Proteine können diese Sequenzen oder Teilsequenzen hieraus enthalten, aber auch aus diesen Sequenzen oder Teilsequenzen hieraus bestehen.

Im Rahmen dieser Beschreibung wird die Bezeichnung Nifie14 für alle humanen Isoformen, bekannt oder neu, auf Nukleinsäuren- oder Aminosäurenbasis, verwendet. Mit diesen Begriffen mit umfaßt sind auch die im Rahmen dieser Beschreibung offenbarten kurzen Sequenzen, beispielsweise Immunisierungssequenzen. Weiterhin mit umfaßt sind auch Homologe, wobei die Homologie zumindest 80%, vorzugsweise mehr als 90%, höchstvorzugsweise mehr als 95%, beträgt, berechnet mit dem Programm MEGALIGN (DNASTAR LASERGENE) in der zum Zeitpunkt der vorliegenden Anmeldung aktuellen Fassung. Im Falle der Nukleinsäuresequenzen sind auch komplementäre oder allelische Varianten mit umfaßt. Weiterhin sind Sequenzen umfaßt, welche lediglich Teilsequenzen der explizit offenbarten Sequenzen, beispielsweise ein Exon oder mehrere Exons, oder komplementärer Sequenzen hierzu darstellen, mit der Maßgabe, daß diese Teilsequenzen im Falle der Nukleinsäuren eine für eine Hybridisierung mit einer erfindungsgemäßen Nukleinsäure hinreichende Länge, zumindest 50 Basen, aufweisen und im Falle der Proteine bzw. Peptide mit zumindest gleicher Affinität an ein protein- oder peptidspezifisches Zielmolekül binden. Weiterhin sind alle mit erfindungsgemäßen Nukleinsäuren hybridisierende Nukleinsäuren umfaßt, nämlich solche, die unter stringenten Bedingungen (5°C bis 25°C unterhalb der Aufschmelztemperatur; siehe ergänzend J.M. Sambrook et al., A laboratory manual, Cold Spring Harbor Laboratory Press (1989) und E.M. Southern, J Mol Biol, 98:503ff (1975)) hybridisieren. Es versteht sich, daß die Erfindung auch Expressionskassetten umfaßt, i.e. eine oder mehrere der erfindungsgemäßen Nukleinsäuresequenzen mit mindestens einer operativ verbundenen Kontroll- oder regulatorischen Sequenz. Eine solche Expressionskassette kann auch eine Sequenz für ein bekanntes Protein umfassen, wobei im Zuge der Translation ein Fusionsprotein aus einem bekannten Protein und einem erfindungsgemäßen Protein oder Peptid entsteht. Ebenso sind auch antisense Sequenzen zu den vorstehenden Nukleinsäuresequenzen umfaßt. Schließlich sind RNA sowie damit korrelierende DNA und umgekehrt umfaßt, ebenso wie genomische DNA als auch korrelierte cDNA und umgekehrt.

Im Zusammenhang mit erfindungsgemäßen Verwendungen umfassen die Begriffe der Nifie14 Nukleinsäuren oder Protein bzw. Peptide neben den Volllängen der offenbarten Sequenzen (siehe auch vorstehender Absatz) auch Teilsequenzen hieraus, und zwar mit einer Mindestlänge von 12 bis 30 Nukleotiden, vorzugsweise 30 bis 90 Nukleotiden, im Falle der Nukleinsäuren und einer Mindestlänge von 4 bis 10 Aminosäuren, vorzugsweise 10 bis 30 Aminosäuren, im Falle der Peptide oder Proteine. Diese Teilsequenzen können in ansonsten von Nifie14 verschiedene Nukleinsäuren- oder Protein- bzw. Peptidsequenzen eingebaut sein.

Der Begriff der Behandlung umfaßt auch die Prophylaxe, insbesondere die Prophylaxe der Progression zu Tumoren.

Als Inhibitor ist eine Verbindung oder Substanz bezeichnet, welche entweder die Bildung von Nifie14 Protein inhibiert oder gebildetes Nifie14 Protein in der Aktivität reduziert, bezogen auf die Nifie14 Aktivität in Abwesenheit des Inhibitors. Insofern kann ein Inhibitor einerseits eine Substanz sein, welche in der Entstehungskaskade von Nifie14 inhibierend eingreift. Auf der anderen Seite kann ein Inhibitor eine Substanz sein, welche mit gebildetem Nifie14 eine Bindung eingeht, und zwar dergestalt, dass weitere physiologische Wechselwirkungen mit endogenen Substanzen zumindest reduziert sind.

Mimikry-Moleküle sind Verbindungen, die den variablen Bereich, insbesondere den Bindungsbereich eines Antikörpers, nachbilden und an gleicher Stelle eines Zielmoleküls binden, wie der zu Grunde liegende Antikörper.

Der Begriff der Antikörper umfaßt polyklonale Antikörper, monoklonale Antikörper, nicht-humane, humane und humanisierte Antikörper, sowie Phage-Display-Antikörper, aber auch chimäre Antikörper sowie spezifische Fragmente der leichten und/oder der schweren Kette des variablen Bereiches zu Grunde liegender Antikörper vorstehender Art sowie anti-idiotypische Antikörper. Die Herstellung bzw. Gewinnung solcher Antikörper mit vorgegebenen Immunogenen ist dem Durchschnittsfachmann wohl vertraut und braucht nicht näher erläutert zu werden. Weiterhin umfaßt der Begriff der Antikörper bispezifische Antikörper. Bispezifische Antikörper kombinieren eine definierte Immunzellaktivität mit einer spezifischen Tumorzellerkennung, wodurch Tumorzellen getötet werden. Ein bispezifischer Antikörper bindet einerseits an ein Auslösemolekül der Immun-Effektorzelle (z.B. CD3, CD16, CD64) und andererseits an Antigene der Tumorzielzelle.

Die galenische Herrichtung einer erfindungsgemäßen pharmazeutischen Zusammensetzung kann in fachüblicher Weise erfolgen. Als Gegenionen für ionische Verbindungen kommen beispielsweise Na+, K+, Li+ oder Cyclohexylammonium infrage. Geeigente feste oder flüssige galenische Zubereitungsformen sind beispielsweise Granulate, Pulver, Dragees, Tabletten, (Mikro-) Kapseln, Suppositorien, Sirupe, Säfte, Suspensionen, Emulsionen, Tropfen oder injizierbare Lösungen (i.v., i.p., i.m.) sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als Hilfsstoffe sei Magnesiumcarbonat, Titandioxyd, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass mindestens ein erfindungsgemäß verwendeter Inhibitor in definierter Dosis mit einem pharmazeutisch geeigneten und physiologisch verträglichen Träger und ggf. weiteren geeigneten Wirk-, Zusatz- oder Hilfsstoffen mit definierter Inhibitordosis gemischt und zu der gewünschten Darreichungsform hergerichtet ist.

Tumorzellen exprimieren Nifie14 differenziell, wenn Normalzellen des gleichen Gewebetyps (des gleichen oder verschiedener Probanden) dieses nicht exprimieren. Tumorzellen überexprimieren Nifie14 spezifisch bzw. differenziell, wenn Nifie14 im Vergleich zu Normalzellen des gleichen Gewebetyps zumindest in doppelter Menge exprimiert wird.

Zytotoxische Komponenten bzw. Gruppen sind Verbindungen, welche direkt oder indirekt Apoptose einleiten bzw. zu Nekrose führen oder zumindest wachstumshemmend wirken. Solche Gruppen bzw. Verbindungen können neben Radioisotopen (z.B. 188Re, 213Bi, 99mTc, 90Y, 131J, 177Lu) insbesondere Zytostatika sein, welche in der Tumortherapie eingesetzt werden. Beispiele hierfür sind: Alkylantien (z.B. Mechlorethamin, Ifosfamid, Chlorambucil, Cyclophosphamid, Melphalan, Alkylsulfonate, Busulphan, Nitrosoharnstoffe, Carmustin, Lomustin, Semustin, Triazene, Dacarbazin), Antimetaboliten (z.B. Folsäure-Antagonisten, Methotrexat, Pyrimidin-Analoga, Fluoruracil, Fluordesoxyuridin, Cytarabin, Gemcitabin, Purin-Analoga, Mercaptopurin), Mitosehemmer (z.B. Vincaalkaloide, Voncristin, Vinblastin, Paclitaxel, Docetaxel, Protaxel), Epipodophyllotoxine (z.B. Etoposid, Teniposid), Antibiotika (z.B. Dactinomycin, Daunorubicin, Idarubicin, Anthracycline, Bleomycin, L-Asparaginase), Platinkomplexverbindungen (z.B. Cisplatin), Hormone und verwandte Verbindungen (z.B. Nebennierenrindensteroide, Aminogluthetimid, Gestagene, Östrogene, Androgene, Antiöstrogene, Tamoxifen, Steriodanaloga, Flutamid). Bei Bindung einer solchen Verbindung mit einer an Nifie14 bindenden Substanz erfolgt die Kopplung dergestalt, daß die Affinität zu Nifie14 um nicht mehr als 90%, vorzugsweise 50%, bezogen auf die Substanz ohne zytostatische Gruppe, reduziert ist und die zytostatische Wirkung der Gruppe um nicht mehr als 90%, vorzugsweise 50%, bezogen auf die Verbindung ohne Substanz, reduziert ist.

Eine immunstimulierende Komponente ist meist ein Protein oder ein wirksamer Bestandteil hiervon, welches Zellen des Immunsystems stimuliert. Beispiele hierfür sind: Zytokine, wie M-CSF, GM-CSF, G-CSF, Interferone, wie IFN-alpha, -beta, -gamma, Interleukine wie IL-1 bis -16 (außer-8), human LIF, Chemokine wie Rantes, MCAF, MIP-1-alpha, -beta, NAP-1 und IL-8.

Eine Reportergruppe ist ein Atom, Molekül oder eine Verbindung, welche in Verbindung mit einem hierauf abgestellten Assay den Nachweis der Reportergruppe und der somit mit der Reportergruppe verbundenen Verbindung oder Substanz ermöglicht. Beispiele für Reportergruppen und hiermit assoziierte Detektionsmethoden sind: 32P-Labeling und Intensitätsmessung mittels Phosphoimager. Viele weitere Beispiele sind dem Durchschnittsfachmann bekannt und bedürfen nicht der detaillierten Aufzählung.

Eine an Nifie14 bindende Substanz kann eine Substanz sein, welche an ein Nifie14 Protein oder eine Nifie14 RNA bindet.

Im Rahmen der vorstehenden Definition gegenüber dem engen Wortsinn erweiterte Begriffsbestimmungen umfassen auch die bestimmten Begriffe im engen Wortsinn.

### Beispiele.

Im Folgenden wird die Erfindung anhand von lediglich bevorzugte Ausführungsformen darstellenden Beispielen und Figuren näher erläutert.

Es zeigen:

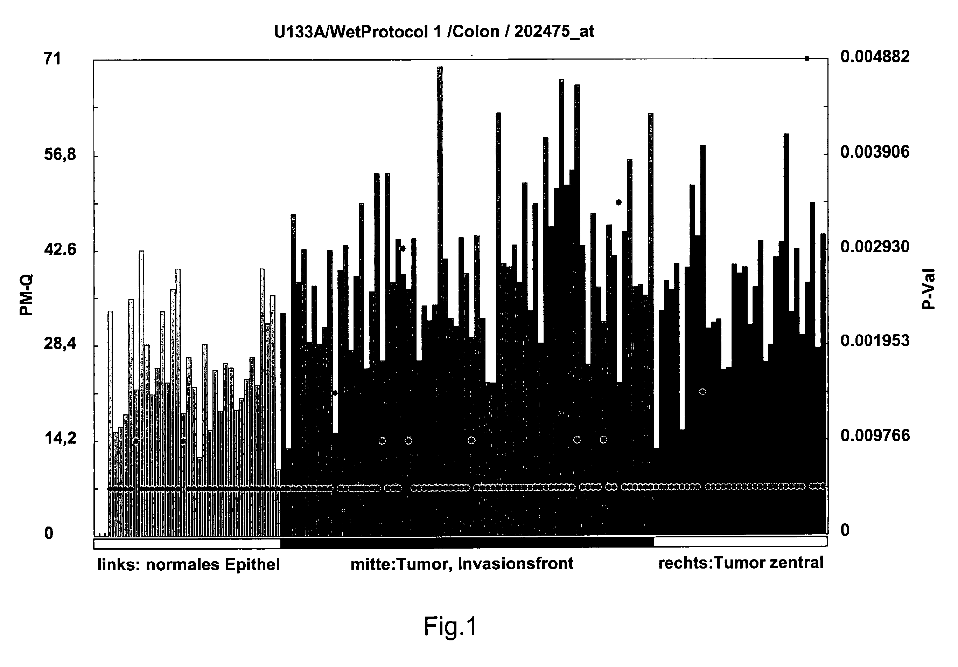

Fig. 1: Chipanalyse zur differentiellen Expression von Nifie14 in Kolontumorgewebe,

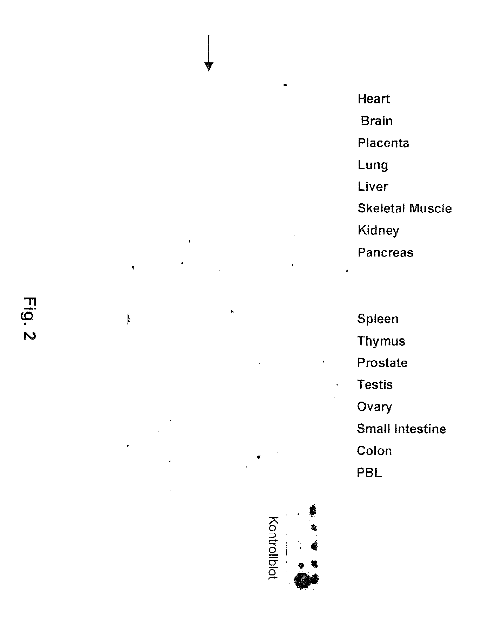

Fig. 2: Nifie14 Expression in Normalgeweben,

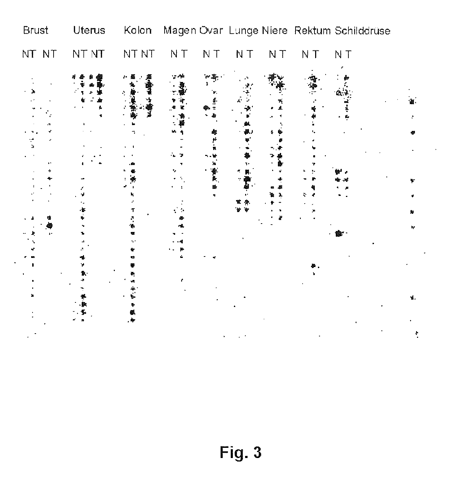

Fig. 3: Krebsgewebe Profiling (CPA),

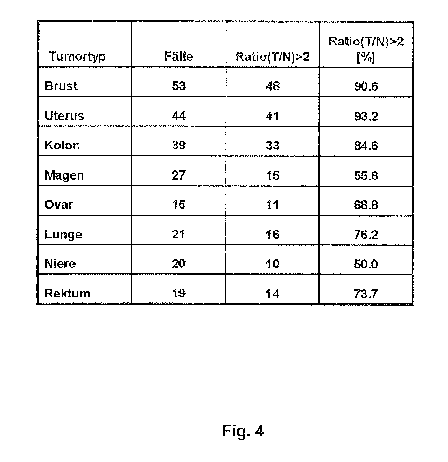

Fig. 4: Tabellarische Auswertung des Profiling (CPA) von Fig. 3,

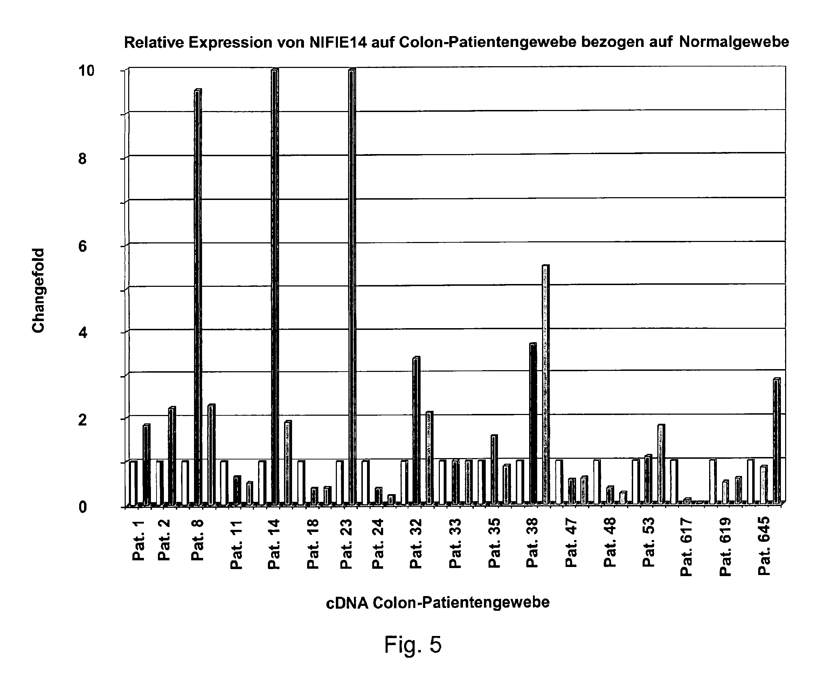

Fig. 5: Taqman Resultate zur Nifie14 Expression in Kolontumoren, bezogen auf zugeordnetes Normalgewebe,

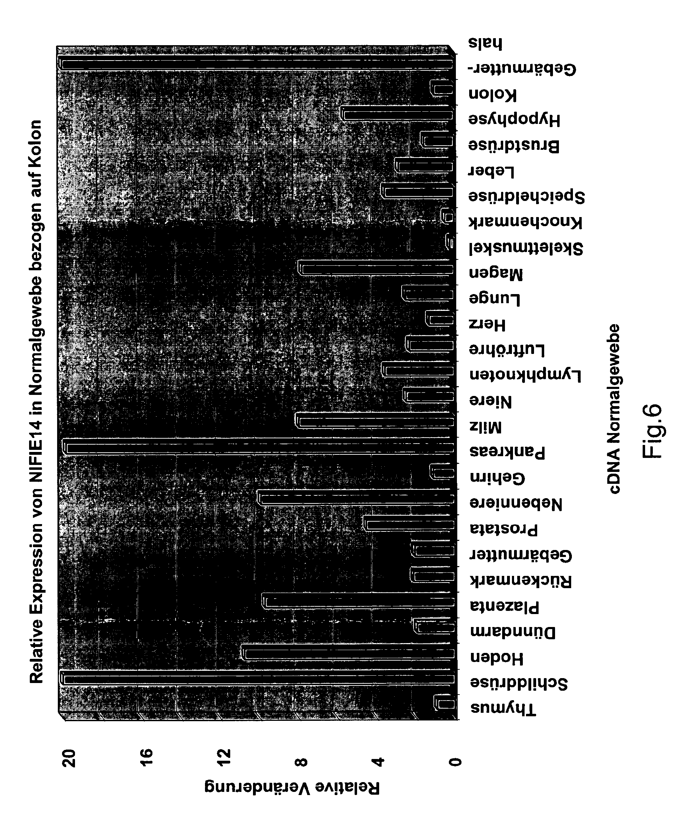

Fig. 6: Taqman Resultate zur Nifie14 Expression in verschiedenen Normalgeweben, bezogen auf Kolon Normalgewebe,

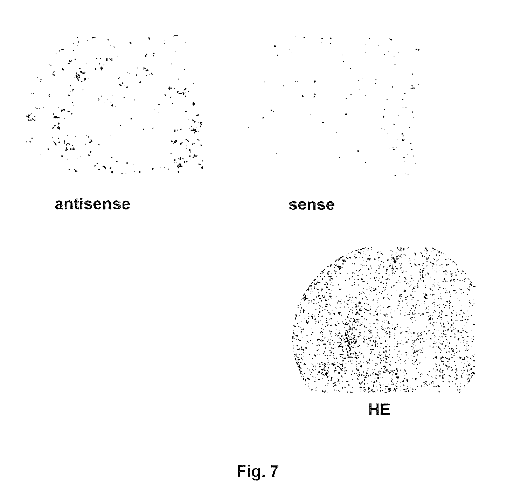

Fig. 7: in situ Hybridisierung von Kolon Tumorgeweben.

### Beispiel 1: Untersuchte Gewebeproben

Es wurde Kolontumorgewebe von Patienten mit zum Zeitpunkt der Erhebung invasiven Tumoren entnommen, und zwar einerseits aus dem Zentraltumor und andererseits aus der Invasionsfront. Zu Vergleichszwecken wurde zugleich Normalgewebe den Patienten entnommen. Die drei Gewebeprobentypen (Kerntumor, Invasionsfront, Normalgewebe) aus jeweils einem Patienten wurden einander zugeordnet. Im Einzelnen wurde wie folgt verfahren. Die Tumor- und -normalgewebeproben wurden gefroren und in 10µm Proben geschnitten. Aus jedem Patienten wurden zumindest 30 Proben gewonnen. Normale und maligne Bereiche wurden durch einen Pathologen mit Hilfe eines Mikroskopes identifiziert und markiert. Hierbei wird ggf. auch das Stadium bzw. die Tumorsubklasse bestimmt und der Probe zugeordnet. Die jeweiligen Bereiche wurden unter dem Mikroskop resektiert unter Verwendung einer Nadel und jeweils separat auf -80°C eingefroren in 150µl GTC Puffer enthaltend 2% β-Mercaptoethanol.

Für das Cancer-Profiling-Array wurden Tumor- und Normalgewebeproben aus Brust (BR), Uterus (UT), Kolon (CO), Magen (ST), Ovar (OV), Lunge LU), Niere (KI), Rektum (RE) und Schilddrüse (TH) verwendet.

### Beispiel 2: Expressionsprofile der untersuchten Gewebe

Die Proben aus Beispiel 1 wurden einer Expressionsanalyse auf Nifie14 mittels der GeneChip-Technologie (Affymetrix) unterworfen. Dabei wird aus Proben aus Beispiel 1 RNA isoliert, amplifiziert und markiert. Die so erhaltene RNA wird einem Genchip aufgegeben, welcher eine Vielzahl von verschiedenen Oligonukleotiden enthält, wobei jeweils eines (oder auch mehrere, zu Kontrollzwecken) für ein definiertes Gen repräsentativ ist, i.e. eine charakteristische Teilsequenz hieraus aufweist. Man erhält sowohl qualitative, wie auch quantitative Information, ob eine betreffende Normal- und/oder Tumorprobe ein betreffendes Gen exprimiert, und zwar auch im Verhältnis Tumor/Normal. Die Analyse ergab eine gegenüber dem Normalgewebe höhere Expression von Nifie14 sowohl in der Invasionsfront des Tumors als auch im Kolon Adenokarzinom selbst (die Höhe eines Balkens entspricht dem mRNA Expressionsniveau). Dies ist in der Fig. 1 dargestellt. Von jedem Patienten wurde jeweils normales Kolongewebe (weiße Balken, links), Kolon Tumorgewebe (schwarze Balken, rechts) und invasive Front des Tumors (graue Balken, mitte) analysiert. In vielen Fällen ist in diesen Proben die Nifie14 RNA Expression der Tumorproben bzw. der invasiven Front mindestens doppelt so hoch, wie in dem zugehörigen Normalgewebe desselben Patienten.

In der Figur 5 ist die relative Expression des Nifie14 in Kolontumoren aus der Invasionsfront (IT) und dem Resttumor (TR) verglichen mit normalem Kolonepitheldargestellt. Gemessen wurde mittels quantitativer PCR (Taqman). Für jeden Patienten liegen drei Messungen der Transkriptmenge vor: normales Epithel (weisse Balken), Invasionsfront (schwarze Balken), und zentraler Tumor bzw. Resttumor (graue Balken). In 8 von 18 Patienten finden sich Expressionverhältnisse T/N > 2.

### Beispiel 3: Expression von Nifie14 in verschiedenen Normalgeweben.

In der Figur 2 sind Ergebnisse von MTN Experimenten zur Expression von Nifie14 in verschiedenen Normalgeweben dargestellt. Nifie14 mRNA ist als schwarze Bande nur in der Pancreas Spur festzustellen. Der Kontrollblot zeigt eine schwarze Färbung, was die Eignung der verwendeten Sonde belegt. Das Nifie14 Transkript ist folglich in Normalgeweben allenfalls schwach exprimiert, in Pancreas noch am stärksten.

Figur 6 zeigt Ergebnisse der relativen Expression in verschiedenen humanen Normalgeweben verglichen mit normalem Kolongewebe. Gemessen wurde analog der Figur 5. Im Vergleich zu Kolon erkennt man sehr hohe Expression in der Schilddrüse, in Pancreas, und im Gebärmutterhals. Es sei hierbei aber anzumerken, dass selbst die in diesem Vergleich hohe Expression in Pancreas noch unter der Expression in Tumorgeweben liegt.

### Beispiel 4: Überexpression von Nifie14 in verschiedenen Normal- und Tumorgeweben.

Ein Cancer Profiling Array (CPA, Clontech) mit 240 aufgetragenen humanen cDNAs von Tumor- und korrespondierenden Normalgeweben jeweils eines Patienten wurde unter Verwendung einr 32P-markierten Nifie14-spezifischen Sonde unter Einsatz eines Phosphoimagers analysiert. Im Einzelnen wurden die Proben aus Beispiel 1 untersucht.

Der Figur 3, welche einen Northern Blot anhand des Cancer-Profiling-Arrays darstellt, entnimmt man, dass eine differenzielle Expression in nahezu allen untersuchten Tumorgeweben (T=Tumor, N=Normal). Oben rechts ist eine Labelling Kontrolle für die Hybridisierung wiedergegeben; 1 pg Nifie14 plasmid DNA wurde mittels der gelabelten Probe problemlos detektiert. Tabelle 1 quantifiziert die Ergebnisse der Figur 2. Dabei wurde eine differenzielle Expression (pos) angenommen bei zumindest 2-facher Überexpression von Nifie14 in Tumorgewebe gegenüber Normalgewebe.

**Tabelle 1**

| Tumor | Überexpression abs. | Überexpression rel. |
|---|---|---|
| Pos Anzahl/Gesamtanz. (%) | | |
| Brust | 48/53 | 90,6 |
| Uterus | 41/44 | 93,2 |
| Kolon | 33/39 | 84,6 |
| Lunge | 16/21 | 76,2 |
| Ovar | 11/16 | 68,8 |
| Niere | 10/20 | 50,0 |
| Magen | 15/27 | 55,6 |
| Rektum | 14/19 | 73,7 |

### Beispiel 4: Immunhistochemischer Nachweis von Tumorzellen oder Nachweis mittels in situ Hybridisierung.

Gewebe wird aus einem Patienten mit Krebs oder dem Verdacht auf Krebs isoliert und als Paraffin- bzw. Gefrierschnitte präpariert. Diese Schnitte werden für den immunhistochemischen Nachweis mit einem gegen ein erfindungsgemäßes Protein gerichteten Antikörper auf die Überexpression des Proteins in Zellen untersucht. Es kann sich beispielsweise um polyklonale Antikörper handeln, die mittels einer der folgenden Peptide als Immunogen erhalten wurden.

VWMITRYDLYHTFR (Pos. 153-166)

LVMSRNAGKGEYKIM (Pos. 86-100)

SGLSEYNAFWKCVQAG (Pos. 24-39)

FFPTWEGGIYDFIGEF (Pos. 56-71)

SRNAGKGEYKI (Pos. 89-99)

RGIEFDWKYIQMSIDS (Pos. 124-139)

VWMITRYDLYHIFRPA (Pos. 153-168)

LCSLGSWAALLARA (Pos. 189-202)

Es können auch Teilsequenzen aus vorstehenden Sequenzen mit zumindest 8 Aminosäuren eingesetzt werden. Die Positionsangaben beziehen sich auf Seq.-ID 2.

Ergebnisse einer in situ Hybridisierung sind in der Figur 7 dargestellt. Gewebeproben aus Colongewebe wurden mit einer Nifie14 spezifischen antisense Sonde inkubiert und mit BM-Purple gefärbt. Die Gegenfärbung erfolgte mit Kernecht-Rot. Es sind maligne und nichtmaligne Epithelzellen voneinander zu unterscheiden, von denen die malignen Zellen eine starke Anfärbung der RNA in der in situ Hybridisierung zeigen ("antisense"). Man erkennt folglich, dass Nifie14 im Colontumorzellen auf RNA Ebene deutlich überexprimiert ist (gefärbte Bereiche in "antisense"), verglichen zur Kontrolle ("sense"), welche eine nur schwache Färbung zeigt. Das Bild "HE" zeigt eine Hämatoxilin/Eosin-Färbung des entsprechenden Tumorareals und zeigt, dass es sich um Kolontumorzellen handelt.

### Beispiel 5: Erzeugung von anti-idiotypischen monoklonalen Antikörpern zu therapeutischen Zwecken

Ausgehend von einem erfindungsgemäß verwendeten Protein oder Teilprotein wird in fachüblicher Weise ein monoklonaler Antikörper Ab1 erzeugt, welcher in der Lage ist, das Protein spezifisch zu erkennen und daran zu binden. Dabei ist es unwesentlich, ob eine funktionale Domäne oder ein anderer zugänglicher Bereich erkannt wird. Mit Hilfe des erzeugten Antikörpers Ab1 wird in ebenso fachüblicher Weise ein zweiter anti-idiotypischer nicht humanisierter, beispielsweise Maus, monoklonaler Antikörper aAB1 erzeugt, welcher zur Herstellung einer pharmazeutischen Zusammensetzung zur Behandlung von Krebs, insbesondere der vorstehend genannten Tumoren, geeignet ist. Die Funktion des Antikörpers aAB1 beruht dabei darauf, dass dieser dem humanen Immunsystem ein Image des (humanen) Protein-Antigens gleichsam vortäuscht, wobei das Immunsystem den Antikörper aAB1 aufgrund seiner mangelnden Humanisierung als körperfremd erkennt. Der humane Körper bildet folglich eigene Antikörper, die gegen aAB1 und somit auch gegen das humane Protein bzw. dieses exprimierende Tumorzellen gerichtet sind.

### Beispiel 6: Nifie14 mRNA Knock-down durch RNAi oder antisense Oligos

Transfektion von Nifie14 spezifischen antisense-Oligonukleotiden oder siRNA-Molekülen in Zellen vermindert die intrazelluläre Menge an Nifie14 mRNA. Tumorzellen werden mit einem geeigneten Oligo bzw. siRNA Molekül transfiziert, nach 48 bis 72 Stunden werden die Zellen lysiert, RNA präpariert und die Menge an Nifie14 mRNA mittels Real-Time PCR Analyse quantifiziert. Die Transfektion eines Nifie14 spezifischen Ologinukleotides bewirkt eine Verringerung der Nifie14 RNA Menge, verglichen mit der Kontrolle, welche mit einem unspezifischen Oligo transfiziert wurde. Wirksame Oligos sind dann therapeutisch einsetzbar im Rahmen geeignet hergerichteter pharmazeutischer Zusammensetzungen.

### Sequence Listing FreeText

SEQUENCE LISTING

<110> Hinzmann, Bernd
      Staub, Eike
      Rosenthal, André
      Hermann, Klaus
      Heiden, Esmeralda
      Dahl, Edgar
      Klaman, Irina
      Mönch, Malve
      Stei, Martin

<120> Verwendung von Niefiel4 bindenden Substanzen zur Diagnose und
      Behandlung von Krebs

<130> MET/DE/0333

<140> DE 103 45 010.6-41
<141> 2003-09-22

<160> 2

<170> PatentIn version 3.2

<210> 1
<211> 869
<212> DNA
<213> Homo sapiens

<400> 1
gccctggccg tggcgaaaga gccggcggag ccggagaccc gctcccggag acgccgcctc      60

gcgatccccg cgcgggcggg accgggcggc cggcatcatg accctgtttc acttcgggaa     120

ctgcttcgct cttgcctact tcccctactt catcacctac aagtgcagcg gcctgtccga     180

gtacaacgcc ttctggaaat gcgtccaggc tggagtcacc tacctctttg tccaactctg     240

caagatgctg ttcttggcca ctttctttcc cacctgggaa ggcggcatct atgacttcat     300

tggggagttc atgaaggcca gcgtggatgt ggcagacctg ataggtctaa accttgtcat     360

gtcccggaat gccggcaagg gagagtacaa gatcatggtt gctgccctgg gctgggccac     420

tgctgagctt attatgtccc gctgcattcc cctatgggtc ggagcccggg gcattgagtt     480

tgactggaag tacatccaga tgagcataga ctccaacatc agtctggtcc attacatcgt     540

cgcgtctgct caggtctgga tgataacacg ctatgatctg taccacacct tccggccagc     600

tgtcctcctg ctgatgttcc tcagtgtcta caaggccttt gttatggaga ccttcgtcca     660

cctctgctcg ctgggcagtt gggcagctct actggcccga gcagtggtaa cggggctgct     720

ggccctcagc actttggccc tgtatgtcgc cgttgtcaat gtgcactcct aggcttggtg     780

tctcagacat tgatgtacct tttccctgcc tcgctccagg ttttagtgaa gtaaacagta     840

tttggaaagt taaaaaaaaa aaaaaaaaa                                       869

<210> 2
<211> 224

<212>  PRT
<213>  Homo sapiens

<400>  2

Met Thr Leu Phe His Phe Gly Asn Cys Phe Ala Leu Ala Tyr Phe Pro
1               5                   10                  15

Tyr Phe Ile Thr Tyr Lys Cys Ser Gly Leu Ser Glu Tyr Asn Ala Phe
            20                  25                  30

Trp Lys Cys Val Gln Ala Gly Val Thr Tyr Leu Phe Val Gln Leu Cys
        35                  40                  45

Lys Met Leu Phe Leu Ala Thr Phe Phe Pro Thr Trp Glu Gly Gly Ile
    50                  55                  60

Tyr Asp Phe Ile Gly Glu Phe Met Lys Ala Ser Val Asp Val Ala Asp
65                  70                  75                  80

Leu Ile Gly Leu Asn Leu Val Met Ser Arg Asn Ala Gly Lys Gly Glu
                85                  90                  95

Tyr Lys Ile Met Val Ala Ala Leu Gly Trp Ala Thr Ala Glu Leu Ile
            100                 105                 110

Met Ser Arg Cys Ile Pro Leu Trp Val Gly Ala Arg Gly Ile Glu Phe
        115                 120                 125

Asp Trp Lys Tyr Ile Gln Met Ser Ile Asp Ser Asn Ile Ser Leu Val
    130                 135                 140

His Tyr Ile Val Ala Ser Ala Gln Val Trp Met Ile Thr Arg Tyr Asp
145                 150                 155                 160

Leu Tyr His Thr Phe Arg Pro Ala Val Leu Leu Leu Met Phe Leu Ser
                165                 170                 175

Val Tyr Lys Ala Phe Val Met Glu Thr Phe Val His Leu Cys Ser Leu
            180                 185                 190

Gly Ser Trp Ala Ala Leu Leu Ala Arg Ala Val Val Thr Gly Leu Leu
        195                 200                 205

Ala Leu Ser Thr Leu Ala Leu Tyr Val Ala Val Val Asn Val His Ser
    210                 215                 220

>SEQ ID 1

GCCCTGGCCGTGGCGAAAGAGCCGGCGGAGCCGGAGACCCGCTCC

CGGAGACGCCGCCTCGCGATCCCCG

CGCGGGCGGGACCGGGCGGCCGGCATCATGACCCTGTTTCACTTCG

GGAACTGCTTCGCTCTTGCCTACT

TCCCCTACTTCATCACCTACAAGTGCAGCGGCCTGTCCGAGTACAAC

GCCTTCTGGAAATGCGTCCAGGC

TGGAGTCACCTACCTCTTTGTCCAACTCTGCAAGATGCTGTTCTTGGC

CACTTTCTTTCCCACCTGGGAA

GGCGGCATCTATGACTTCATTGGGGAGTTCATGAAGGCCAGCGTGG

ATGTGGCAGACCTGATAGGTCTAA

ACCTTGTCATGTCCCGGAATGCCGGCAAGGGAGAGTACAAGATCATG

GTTGCTGCCCTGGGCTGGGCCAC

TGCTGAGCTTATTATGTCCCGCTGCATTCCCCTATGGGTCGGAGCCC

GGGGCATTGAGTTTGACTGGAAG

TACATCCAGATGAGCATAGACTCCAACATCAGTCTGGTCCATTACATC

GTCGCGTCTGCTCAGGTCTGGA

TGATAACACGCTATGATCTGTACCACACCTTCCGGCCAGCTGTCCTC

CTGCTGATGTTCCTCAGTGTCTA

CAAGGCCTTTGTTATGGAGACCTTCGTCCACCTCTGCTCGCTGGGCA

GTTGGGCAGCTCTACTGGCCCGA

GCAGTGGTAACGGGGCTGCTGGCCCTCAGCACTTTGGCCCTGTATG

TCGCCGTTGTCAATGTGCACTCCT

AGGCTTGGTGTCTCAGACATTGATGTACCTTTTCCCTGCCTCGCTCCA

GGTTTTAGTGAAGTAAACAGTA

TTTGGAAAGTTAAAAAAAAAAAAAAAAA

>SEQ ID 2

MTLFHFGNCFALAYFPYFITYKCSGLSEYNAFWKCVQAGVTYLFVQLCK

MLFLATFFPTWEGGIYDFIGE

FMKASVDVADLIGLNLVMSRNAGKGEYKIMVAALGWATAELIMSRCIPLW

VGARGIEFDWKYIQMSIDSN

ISLVHYIVASAQVWMITRYDLYHTFRPAVLLLMFLSVYKAFVMETFVHLCS

LGSWAALLARAVVTGLLAL

STLALYVAVVNVHS

## Patentansprüche

1. Verwendung einer für ein Nifie14 Peptid oder Protein codierenden Nukleinsäure und/oder eines Nifie14 Peptids oder Proteins zur Detektion von Krebs oder zur Detektion eines Risikos der Erkrankung an einem Tumor, wobei eine Gewebeprobe auf Transkription oder Übertranskription von Nifie14 RNA oder auf Expression oder Überexpression eines Nifie14 Proteins untersucht wird.

2. Verwendung nach Anspruch 1, wobei eine an für Nifie14 codierende Nukleinsäure oder eine an Nifie14 Protein oder Peptid bindende Detektorsubstanz, vorzugsweise enthaltend eine Reportergruppe, verwendet wird, wobei Bindung besagter Nukleinsäure und/oder besagten Proteins oder Peptids an die Detektorsubstanz halbquantitativ oder quantitativ detektiert wird.

3. Verwendung einer Nifie14 RNA oder eines Nifie14 Proteins oder Peptids zum Screenen nach daran bindenden Substanzen, insbesondere nach prospektiven Wirkstoffen zur Inhibierung von besagter RNA oder besagtem Protein oder Peptid oder nach prospektiven Detektorsubstanzen, wobei eine prospektive Substanz oder eine Mischung solcher prospektiver Substanzen mit besagter RNA oder besagtem Protein oder Peptid kontaktiert wird, wobei mit einem Bindungsassay Bindungsereignisse festgestellt werden, und wobei eine bindende prospektive Substanz, ggf. nach Dekonvolutierung, selektiert wird.

4. Verwendung einer Nifie14 modulierenden, inhibierenden oder daran bindenden Substanz zur Herstellung einer pharmazeutischen Zusammensetzung zur Behandlung von Krebs oder zur Herstellung einer pharmazeutischen Zusammensetzung zur Diagnose eines Tumors oder zur Diagnose eines Progressionsrisikos eines Tumors.

5. Verwendung nach Anspruch 4, wobei die Substanz ein vorzugsweise monoklonaler Antikörper ist, welcher beispielsweise durch Immunisierung eines nicht-menschlichen Säugetiers mit einem Nifie14 Peptid oder Protein, mit Nifie14 transfizierten Zellen oder Membranvesikeln solcher Zellen, oder einer hierfür für codierenden cDNA, erhältlich ist, oder ein Phage-Display Antikörper ist.

6. Verwendung nach Anspruch 4, wobei die Substanz eine Mimikriverbindung eines Antikörpers gegen ein Nifie14 Peptid oder Protein ist.

7. Verwendung nach Anspruch 4, wobei die Substanz, ein Aptamer, eine antisense RNA, eine siRNA, oder ein Ribozym ist.

8. Verwendung nach einem der Ansprüche 4 bis 7, wobei die Substanz zusätzlich eine zytotoxische und/oder immunstimulierende Komponente trägt.

9. Verwendung nach einem der Ansprüche 4 bis 8, wobei die pharmazeutische Zusammensetzung zur lokalen Applikation und/oder systemischen Applikation in Tumorzellen enthaltendem Gewebe hergerichtet ist.

10. Verfahren zur Diagnose einer Krebserkrankung, oder des Risikos der Erkrankung an einem solchen Tumor, wobei eine an Nifie14 bindende Detektorsubstanz in einer Ausführungsform mit einer Reportergruppe in zu untersuchendes Gewebe appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als Tumorzellen enthaltend oder als erkrankungsgefährdet qualifiziert wird.

11. Verfahren zur Behandlung von Krebs, wobei eine pharmazeutische Zusammensetzung nach einem der Ansprüche 4 bis 9 in einer physiologisch wirksamen Dosis und galenisch für die anzuwendende Darreichungsform hergerichtet einem Patienten dargereicht wird.

12. Protein oder Peptid enthaltend oder bestehend aus einer Sequenz Seq.-ID 2, oder einer Teilsequenz der Mindestlänge von 4, 5, 6, 7, 8, 9, oder 10 AS aus besagten Sequenzen, oder Nukleinsäure codierend für ein vorstehend definiertes Protein oder Peptid, insbesondere enthaltend eine Teilsequenz von 12, 15, 18, 21, 24, 27, oder 30 Nukleotiden aus einer der Sequenzen SEQ-ID 1.
